# EUROPEAN PATENT APPLICATION

(11) **EP 4 331 577 A1**
(43) Date of publication of application: **06.03.2024**
(21) Application number: 23192527.2
(22) Date of filing: 21.08.2023
(51) Int. Cl.: A61K 31/164

(54) **A NUTRACEUTIC TABLET COMPRISING PALMITOYLETHANOLAMIDE**

(30) Priority: 30.08.2022 IT 202200017820
(71) Applicant: Unifarco S.p.A., 32035 Santa Giustina (BL) (IT)
(72) Inventor: Bonini, Mauro, Istrana TV (IT); Feltrin, Sara, Istrana TV (IT); Callegari, Martina, Istrana TV (IT); Sfriso, Mattia, Istrana TV (IT); Savio, Margherita, Istrana TV (IT); Baratto, Giovanni, Santa Giustina BL (IT); Francescato, Stefano, Santa Giustina BL (IT); Casanova, Elena, Santa Giustina BL (IT)
(74) Representative: Perani & Partners S.p.A.

(57) **Abstract**

Gastro-coated tablet containing palmitoylethanolamide as active ingredient, preferably in quantities equal to 200mg whose core α) is characterized by a friability of less than 1% w/w on the total weight of the tablet measured according to the FUI XII test (2.9.7) and, when coated, it shows a PEA release profile longer than 120 minutes.

## Description

### TECHNICAL FIELD

The present invention concerns a nutraceutic composition comprising as active ingredient palmitoylethanolamide in the form of a gastro-resistant coated tablet.

### BACKGROUND

The intestine represents the most extensive interface between our body and the external environment and is an organ intended for several functions, among which the best known are the digestion and the absorption of nutrients. It is also home to the recruitment and training of the immune system and is to date considered our second brain. But the main role of this complex organ is to act as a physical and immunological barrier. A healthy intestinal barrier is a fundamental condition for the well-being of our body.

The enteric nervous system (ENS) is part, together with the orthosympathetic and parasympathetic nervous system, of the autonomic nervous system (ANS). The enteric nervous system consists of approximately 500 million neurons in humans, which are grouped into two main networks: the submucosal plexus (or Meissner's plexus) located at the level of the submucosal tunica and the mienteric plexus (or Auerbach plexus) located at the level of the muscular tunica. In both nerve plexuses, the cell bodies of the neurons form small aggregates called ganglia, surrounded by the glial cells, with supporting functions. The neuronal projections connect the ganglia to each other and to the intestinal epithelium [K. Walsh e A. Zemper, "The Enteric Nervous System for Epithelial Researchers: Basic Anatomy, Techniques, and Interactions With the Epithelium," Cell Mol Gastroenterol Hepatol, vol. 8, no. 3, pp. 369-378, 2019.].

The submucosal nerve plexus contains sensory neurons that receive (chemical, mechanical or thermal) signals from the intestinal lumen and mucosa, the ENS interneuron network integrates this information and initiates appropriate responses through the submucosal plexus neurons, which coordinate the intestinal secretion, and the mienteric plexus neurons, which control motility. The two nerve plexuses allow the so-called "local reflexes" to start, be integrated and end completely in the gastrointestinal tract. The reflexes that are integrated on site are called "short reflexes". They are opposed by the "long reflexes", which start from the ENS and reach the CNS through the vagus nerve, to be integrated.

The enteroendocrine cells (EEC) represent only 1% of the epithelial population but are essential for the maintenance of homoeostasis as they act as chemosensors, integrating extrinsic and intrinsic signals from the intestinal lumen and from the epithelium and communicating them to the nervous system. Enterochromaffin cells are a subset of EECs important for serotonin production.

The endocannabinoid system (ECS) consists of the endogenous ligands anandamide and 2-arachidonoylglycerol, of the enzymes necessary for their biosynthesis and degradation, and of the receptors for the cannabinoids CB1 and CB2. The endogenous cannabinoid system is an important regulator of the gastrointestinal motility, it is involved in the control of the visceral painful sensations and the rate of inflammation.

Under conditions of chronic stress, the transcription of the gene coding for CB1 is modified through epigenetic processes: this might link stress to visceral pain.

The ECS is also involved centrally in the manifestation of stress, the signals mediated by the endocannabinoids reduce the activity of the hypothalamus-pituitary-adrenal axis through the action in specific regions of the brain (prefrontal cortex, amygdala and hypothalamus).

The endocannabinoids are synthesized from membrane lipids in response to specific signals and are not stored in vesicles as is the case with the neurotransmitters.

The CB 1 and CB2 receptors were identified in the enteric nerves and at the level of the intestinal epithelium, CB1 in particular was identified on the surface of the EEC. Under physiological conditions, the CB1 receptor (coupled to G proteins) seems to be the most active. Its so widespread distribution underlines on the one hand the importance of the endocannabinoid system, but on the other hand it poses several problems of using CB1 as a therapeutic target, since its ligands could exert a wide variety of effects both centrally and peripherally. While CB 1 is a purely neuronal receptor, CB2 is also expressed by the intestinal immune and epithelial cells. Some studies have observed that particular strains of Lactobacillus, administered to mouse models, cause a reduction in visceral sensitivity. However, while some studies record an up-regulation of CB2 exerted by lactobacilli, others have highlighted a down-regulation of the expression of this receptor; therefore, the role of CB2 in the attenuation of visceral pain is still being studied.

CB 1-binding endocannabinoids have been shown to have an analgesic effect on pain signalling and that the inhibitors of the endocannabinoid degradation have been shown to reduce the stress-related hyperalgesia. The increase in the anandamide signalling pathways reduce anxiety and the thermal hyperalgesia induced by chronic stress.

Low doses of CB1 agonists reduce the activity of the hypothalamus-pituitary-adrenal axis, both baseline and stress-induced ones. ECS suppresses the axis activity through distinct actions in different brain regions [K. Sharkey and J. Wiley, "The role of the endocannabinoid system in the brain-gut axis," Gastroenterology, vol. 151, no. 2, pp. 252-266, 2016].

The exogenous cannabinoids are able to reduce the symptoms of colitis and a possible therapeutic strategy is to increase the levels of the endogenous cannabinoids by inhibiting their degradation.

Anandamide acts on CB1, while Palmitoylethanolamide (PEA) and oleoylethanolamide act through the Perosomal Proliferator Activated Receptor (PPARα, a nuclear receptor). PEA is a potent anti-inflammatory agent that reduces the symptoms of colitis in the mouse and the secretion of inflammatory cytokines. PEA binds PPARα and reduces NF-kB-induced inflammation. PEA, like the anandamide, influences the glial cells and the neurons in the intestine through different receptors on each cell type and its action is blocked by the antagonists acting on the PPARα receptor.

Palmitoylethanolamide (PEA) is a lipid mediator belonging to the family of N-acylethanolamines and is characterized by the following formula.

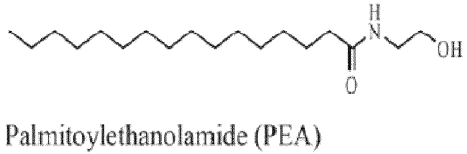

Neuroprotective, anti-inflammatory and analgesic properties are attributed to it. PEA in nature is found in numerous foods such as legumes, egg yolks and peanuts but also in the tissues of animals and human beings. The biosynthesis in the animal kingdom occurs through hydrolysis of the direct phospholipid precursor: N-acyl-phosphatidyl-ethanolamine, process catalysed by the precursor-selective phospholipase. In contrast, degradation of PEA (amide bond breakage) occurs by two different hydrolytic enzymes: fatty acid amide hydrolase (FAAH) and N-acylethanolamine acid amidohydrolase (NAAA). The first prefers anandamide as a substrate to be metabolized; the second, on the other hand, manifests a strong affinity for PEA [S. Petrosino e V. Di Marzo, "The pharmacology of palmitoyletathanolamide and first data on the therapeutic efficacy of some of its new formulations," Br J Pharmacol, vol. 174, no. 11, pp. 1349-1365, 2017].

The PEA seems capable of exerting its beneficial effects through multiple pathways. The first pathway was proposed by Prof. Rita Levi-Montalcini and involves the under-regulation of the mast cell activation through a mechanism called Autacoid Local Injury Antagonism (ALIA). Subsequently, another mechanism directly mediated by at least two receptors was proposed: PPAR-α and GPCR 55. Initially, it was believed that PEA could also represent a CB2 agonist. However, recent studies have shown that the affinity for this receptor is very low, justifying the failure to reduce the anti-inflammatory effect of the PEA in the presence of CB2 antagonists. However, by entourage effect, the PEA could activate the CB 1 and CB2 receptors through inhibition of the FAAH enzyme expression. Similarly, the PEA could activate the TRPV1 (transient receptor potential vanilloid receptor type 1) channels, also targets of the endocannabinoids. Not only that, in 2013, researchers demonstrated the ability of PEA to activate TRPV1 and CB2 channels through the involvement of PPAR-α receptors. What has been said so far indicates that the PEA exerts its effects through multiple pathways, in part, connected to each other.

The analgesic action of the PEA appears to be mediated by the CB1 and PPAR-α receptors and by the TRPV1 channels. In pain conditions, the endogenous expression of PEA decreases and that of arachidonoylglycerol and anandamide increases. However, the increase of the latter two endocannabinoids is not such as to exert an analgesic action due to the reduced levels of PEA production. Therefore, PEA supplementation could represent an effective alternative to potentiate the endogenous anti-nociceptive mechanisms exerted by the endocannabinoids.

A meta-analysis of 10 clinical trials (1298 subjects involved) supports the ability of the PEA to reduce pain perception significantly compared to what was recorded in the control groups. The daily dose used in the included works ranges from 300 mg to 1200 mg, although most studies are around 600 mg. In any case, a meta-regression showed that there is no statistically significant association between PEA dose and reported efficacy. Similarly, the duration of the treatment, varying from 10 to 180 days, does not seem to significantly impact the desired result. In addition, overall, the PEA-based treatment appears to be well tolerated [B. Artukoglu, C. Beyer, A. Zuloff-Shani e al, "Efficacy of Palmitoylethanolamide for Pain: A Meta-Analysis.," Pain Physician, vol. 20, no. 5, pp. 353-362, 2017].

To date, little data are available on the pharmacokinetics and bioavailability of the PEA. The intraperitoneal administration involves the distribution of the active ingredient in different districts, in the following descending order: adrenal gland, diaphragm, spleen, kidneys, testicle, lung, liver, heart, brain, plasma and erythrocytes. By using an in vivo model, Artamonov et al. demonstrated the ability of the PEA to reach the hypothalamus, pituitary, and adrenal gland within 20 minutes from the oral administration. This recording demonstrates the ability of the PEA to cross the blood-brain barrier, although in small quantities due to **the poor** solubility of the active ingredient. In vivo studies demonstrate that the oral administration of PEA, by gastric tube, results in a plasma peak in 15 minutes that corresponds to 20 times the baseline level restored 2 hours after administration. From a clinical study conducted on healthy volunteers, it was found that the oral intake of 300 mg of micronized PEA results in a plasma increase of the active ingredient that peaks at 2 hours after administration. To date, micronized and ultra-micronized forms of PEA are available on the market. It is known, in fact, that the solubility of the active ingredients is intrinsically correlated to the particle size. The reduction of the latter leads to an increase in the surface area and, consequently, in the solubility and bioavailability of the active ingredient.

US 201101171313 and USP6548550 describe processes for preparing formulations in which the PEA is in a micronized form and in an ultra-micronized form.

However, in NL2011448 it was verified that PEA especially in an ultra-micronized form gives rise to undesired metabolites such as for example 2-arachidonoylglycerol which is not essential for the desired treatment. This metabolite present in considerable quantities has affinities for the CB1 and CB2 receptors for which the PEA does not have an affinity; therefore, it can give rise to unwanted side effects,

A further disadvantage in the formulations based on poorly water-soluble PEA lies in the use of lubricants such as magnesium stearate which tends to form films on other excipients during prolonged mixing leading to lamination and adhesion of said lubricant to crospovidone used as a binding agent in the granulation phase, a phenomenon that further slows down the disintegration of the PEA tablets, also slows down the rate of dissolution of said active ingredient.

Another drawback of the PEA formulation of the known art lies in the use of high amounts of sorbitol, which can give rise to diarrhoea and gastrointestinal disorders in sensitive subjects and those suffering from diabetes.

The need is therefore felt to have tablets comprising PEA with adequate bioavailability to manage the non-physiological conditions described above, even if they contain magnesium stearate as a lubricant.

### SUMMARY OF THE INVENTION

The Applicant has now formulated tablets that can pass through the stomach in an intact form and reach the intestine such as to release the active ingredient in an effective amount and in the most appropriate site to exert its effectiveness, as demonstrated in the attached examples

To this end, the Applicant has formulated a tablet wherein the core α) contains dispersed an amount preferably equal to 200 mg of palmitoylethanolamide which is not in an ultra-micronized form nor is it in a micronized form, this core is characterized by a friability of less than 1% w/w on the total weight of the tablet measured according to the FUI XII test (2.9.7) and, when coated, it shows a PEA release profile longer than 120 minutes.

The present invention therefore relates to tablets with gastro-resistant coating, wherein the aforesaid core (α) comprises or consists of:
a) Palmitoylethanolamide
b) at least one diluent selected from: microcrystalline cellulose and pregelatinized starch;
c) a binding agent consisting of hydroxypropylcellulose,
d) at least one inorganic filler selected from: dibasic calcium phosphate dihydrate and calcium carbonate
e) at least one lubricating agent selected from magnesium stearate, precipitated amorphous silica or mixtures thereof;
f) a disintegrating agent consisting of cross-linked sodium carboxymethylcellulose, and wherein:
   I) the palmitoylethanolamide is not in an ultra-micronized form nor is it in a micronized form
   II) the weight ratio of sodium hydroxypropyl cellulose to carboxymethylcellulose is between 4.5 and 10.

The Applicant has in fact demonstrated that the presence of hydroxypropylcellulose as a binding agent (c) and sodium carboxymethylcellulose as a disintegrating agent in the aforesaid weight ratios allows to obtain the aforesaid optimal values in terms of friability of the uncoated tablet and of PEA release in the aforesaid times.

### DETAILED DESCRIPTION OF THE INVENTION

For the purposes of the present invention, the definition "comprising" does not exclude the presence of other components than those expressly mentioned after said definition.

The definition "consisting of" excludes the presence of additional components in addition to those mentioned after this definition.

For the purposes of the present invention, palmitoylethanolamide in an ultra-micronized form is understood as palmitoylethanolamide having the following granulometric characteristics reported in the following table A and derived from the international patent application WO2011/027373.

**TABLE A**

| Particle diameter | micronized palmitoylethanolamide | ultra-micronized palmitoylethanolamide |
|---|---|---|
| > 14 microns | traces | Absent |
| <10 microns | About 96% | 100% |
| <6 microns | 80% | 99.9% |
| <2 microns | Not indicated | 59.6% |
| <1 micron | Not indicated | 14.7% |
| <0.6 micron | Not indicated | 2.0% |

Therefore, the palmitoylethanolamide used in the gastro-resistant tablets object of the invention must have the following characteristics:
particles with sizes greater than 14 microns in quantities comprised between 60 and 100%;
particles with sizes of less than 10 microns in quantities comprised between 1-20%,
particles with sizes of less than 6 microns quantities comprised between 1 and 20%
particles with sizes of less than 2 microns between 0 and 1% and instead the particles with sizes of less than 1 micron and 0.6 microns are absent.

According to a particularly preferred solution, the palmitoylethanolamide particles dispersed in the core α) of the gastro-resistant tablets object of the present invention have a granulometric distribution reported in the following table B

**Table B**

| Particle diameter | **Content in percentage** |
|---|---|
| >14 µm | 76 |
| <10 µm | 16 |
| <6 µm | 8 |
| <2 µm | 0.5 |
| <1 µm | 0 |
| <0.6 µm | 0 |

The weight ratio of the binding agent c) / disintegrating agent f) is preferably 6.9.

Preferably the palmitoylethanolamide a) in the tablets object of the invention is contained in quantities comprised between 23 and 27%, more preferably equal to 25.25% by weight on the total weight of the core.

The diluent b) in the core of the tablet object of the invention is preferably microcrystalline cellulose and is preferably comprised in quantities comprised between 30 and 40%, preferably equal to 34.13%, by weight of the total weight of said core.

The binding agent c) in the core of the tablet object of the present invention, the hydroxypropylcellulose, is preferably present in quantities comprised between 5 and 7.5%, more preferably above 6.5% even more preferably equal to 6.88% by weight on the total weight of said core.

The filler d) in the core object of the present invention is preferably dibasic calcium phosphate preferably in concentrations comprised between 25 and 35% more preferably equal to 30.27% by weight on the total weight of said core.

The lubricating agent e) in the core of the tablet according to the present invention is preferably a mixture of magnesium stearate and precipitated amorphous silica at total concentrations comprised between 2 and 3% preferably equal to 2.26% by weight on the total weight of said core.

The disintegrating agent f) contained in the core of the tablet object of the present invention is preferably cross-linked sodium carboxymethylcellulose in a concentration preferably comprised between 0.5 and 3%, more preferably equal to 1% by weight on the total weight of the core.

In the tablet object of the present invention, the core (α) is coated with a β coating) comprising:
- a first opacifying coating β1) which wraps the aforesaid core comprising or consisting of:
   g) at least one selected film-forming polymer consisting of polyvinyl alcohol,
   h) an opacifying agent selected from calcium carbonate and talc and mixtures thereof;
   i) at least one plasticizer selected from polyethylene glycol, macrogol or mixtures thereof;
- a second gastroprotective coating β2 wrapping the tablet coated with the coating β1, said second coating comprising:
   j) a film-forming polymer selected from ethylcellulose and propyl cellulose:
   k) a plasticizer selected from a mixture of medium chain fatty acid triglycerides, oleic acid or mixtures thereof;
   l) a lubricant selected from stearic acid and its salts
   m) a thickening agent selected from sodium alginate, gum arabic

According to a preferred solution of the tablets object of the invention the coating β1) constitutes between 2.5 and 3.5%, more preferably 3% by weight on the total weight of the core α) and the coating β2) constitutes between 2% and 4%, preferably 2.5% by weight on the weight of the core including the coating β1.

Preferably in the first coating
- the at least one film-forming polymer g) is polyvinyl alcohol at concentrations preferably comprised between 38% and 42%, more preferably 40% by weight on the total weight of said first coating;
- the opacifying agent h) consists of a mixture of calcium carbonate and talc, more preferably at total concentrations comprised between 38% and 42% even more preferably 39.8% by weight on the total weight of said first coating;
- the plasticizer i) is a mixture of PEG and Macrogol more preferably at concentrations comprised between 18% and 22% even more preferably 20.20% by weight on the total weight of said coating.

According to another preferred solution in the second coating β2):
- the film-forming polymer j) ethyl cellulose is present at concentrations comprised between 17.5% and 19.5%, more preferably 18.80% by weight of the total weight of said coating;
- the plasticizer k) is a mixture of triglycerides of medium chain fatty acids and oleic acid at concentrations comprised between 3% and 5%, preferably 4% by weight on the total weight of said second coating;
- the lubricant l) is stearic acid at concentrations comprised between: 0.03% and 0.05% more preferably 0.0423% by weight on the total weight of said second coating;
- the thickening agent m) is sodium alginate at concentrations comprised between 3.5% and 5.5% more preferably it is 4.18% by weight on the total weight of said coating.

The tablet object of the present invention may comprise other active ingredients in the core and according to a particularly preferred solution comprises vitamin B2.

A further object is the process for preparing gastro-resistant tablets object of the present invention, in particular coated respectively with coatings β1 and β2 and comprising the following steps:
i) The components a) -f) are dry mixed;
ii) the mixture from the previous step is compressed;
iii) a suspension in water and/or ethanol of components g), h) and i) is prepared so as to have a total concentration of components g) + h) + i) comprised between 15% and 35% more preferably 20.0% by weight on the total weight of said suspension in water and/or ethanol;
iv) applying the coating β1) by nebulising the suspension at a temperature comprised between 38 °C and 46 °C until the suspension to be nebulized is exhausted;
v) the coated tablet from step iv) is cooled;
vi) the aqueous suspension of components j), k), l), and m) is prepared until a total concentration of j) +k) + l) + m) is obtained between 8% and 15% more preferably 10% by weight of the total weight of the aqueous suspension;
vii) the coating β2) is applied by nebulisation of the aqueous suspension obtained in step vi) at a temperature comprised between 38 °C and 46 °C, and is maintained at such temperature until the suspension to be nebulized is exhausted.

### EXAMPLE 1

The particularly preferred qualitative-quantitative composition of the gastro-coated tablet according to the present invention is reported below in the following table 1.

**TABLE 1-composition of tablet 1 (batch MC032)**

| | Components | g | % by weight |
|---|---|---|---|
| **Core α)** | Vitamin B2, Riboflavin | 0.0018200 | 0.23 |
| | Palmitoylethanolamide (PEA) | 0.20202 | 25.25 |
| | Microcrystalline cellulose - E 460 (i) | 0.273 | 34.13 |
| | Hydroxypropylcellulose E463 | 0.055 | 6.88 |
| | Dibasic calcium phosphate dihydrate E 341 (Ii) | 0.24216 | 30.27 |
| | Cross-linked sodium carboxymethylcellulose E 468 | 0.008 | 1.00 |
| | Magnesium stearate (E470b) | 0.009 | 1.13 |
| | Amorphous silica precipitated E 551 | 0.009 | 1.13 |
| **Total** | | 0.800 | 100 |
| **Coating β1)** | Polyvinyl alcohol, PEG + Macrogol, calcium carbonate * | 0.024 | 3% (weight increase) |
| **Coating β2)** | Sodium alginate and purified stearic acid ** | 0.00309286 | 0.38% (on the total weight of the tablet with first coating) |
| | Ethyl cellulose, medium chain fatty acid triglycerides, oleic acid*** | 0.01750714 | 2.12% (on the total weight of the tablet with first coating) |

| | | | |
|---|---|---|---|
| * Commercial composition used for the coating β1: Nutrafinish white 169F280013 (THIO₂ free), calcium carbonate, polyvinyl alcohol, Macrogol + polyethylene glycol ** Commercial composition used for the coating β2 NS Enteric Clear 29Z19241: Sodium alginate 8133, purified stearic acid. ^{∗∗∗}Commercial composition used for the coating β2 Surelease Clear E-7-19040: purified water, ethylcellulose, ammonium hydroxide, medium chain fatty acid triglycerides, oleic acid. | | | |

### EXAMPLE 2 Preparation of tablet 1

### 2.1 Preparation of core α)

The components of the core are added in the following order after sieving: premix Vitamin B2 and part of the microcrystalline Cellulose; add to the premix Palmitoylethanolamide, Hydroxypropylcellulose, Dibasic Calcium Phosphate Bihydrate, the remaining part of Microcrystalline Cellulose, Cross-linked Sodium Carboxymethylcellulose, Magnesium Stearate, Precipitated Amorphous Silica. The mixture is mixed until a homogeneous mixture is obtained.

The mixture is compressed by applying a pressure between 31kN and 41kN

### 2.2 Application of the opacifying coating β1)

On the tablet obtained in the previous step, the commercial composition used as reported in table 1(*) is applied,

### 2.3 Application of the gastroenteric coating β2)

A mixture of the commercial compositions reported in table 1 whose qualitative composition is reported in (**) and (* *) is applied.

### EXAMPLE 3 Comparative tests

### 3.1. Tablet friability test in accordance with Pharmacopoeia XII 2.9.7.

This test provides guidance for the determination of the friability of uncoated tablets. The measure of the friability is complementary to other measures of mechanical strength, for example those of breaking strength.

### 3.2. Disintegration test in accordance with Pharmacopoeia XII 2.9.1

### For the tablets coated with enteric gastro-resistant coating, the aforesaid disintegration test was carried out.

For the two tests, the following tablets were tested .

**Tablet 1** whose composition is reported in example 1 and was prepared as described in example 2.

**Tablet 2** not coated (batch FL749), whose composition is indicated in the following table 2:

**TABLE 2 -composition of tablet 2**

| Components | g | % by weight |
|---|---|---|
| Palmitoylethanolamide (PEA) | 0.20202 | 25.25 |
| Vitamin B2 | 0.00182 | 0.23 |
| Microcrystalline cellulose - E 460 (i) | 0.276279 | 34.53 |
| Hydroxypropylcellulose E463 | 0.030 | 3.75 |
| Dibasic calcium phosphate dihydrate E 341 (Ii) | 0.250881 | 31.36 |
| Cross-linked sodium carboxymethylcellulose E 468 | 0.023 | 2.88 |
| Magnesium stearate (E470b) | 0.008 | 1 |
| Amorphous silica precipitated E 551 | 0.008 | 1 |

**Tablet 3** not coated (batch MC022) and prepared as described in example 2.1 whose composition is reported in the following table 3

**TABLE 3- composition of tablet 3**

| Components | g | % by weight |
|---|---|---|
| Palmitoylethanolamide (PEA) | 0.20202 | 25.25 |
| Vitamin B2 | 0.00182 | 0.23 |
| Microcrystalline cellulose - E 460 (i) | 0.25016 | 31.27 |
| Hydroxypropylcellulose E463 | 0.030 | 3.75 |
| Hydroxypropyl methylcellulose | 0.050 | 6.25 |
| Dibasic calcium phosphate dihydrate E 341 (Ii) | 0.25 | 31.25 |
| Cross-linked sodium carboxymethylcellulose E 468 | // | // |
| Magnesium stearate (E470b) | 0.008 | 1 |
| Amorphous silica precipitated E 551 | 0.008 | 1 |

**Tablet 4 not coated** (batch MC023) and prepared as described in example 2.1, the composition of which is reported in the following table 4

**TABLE 4-composition of tablet 4**

| Components | g | % by weight |
|---|---|---|
| Palmitoylethanolamide (PEA) | 0.20202 | 25.25 |
| Vitamin B2 | 0.00182 | 0.23 |
| Microcrystalline cellulose - E 460 (i) | 0.22516 | 28.15 |
| Hydroxypropylcellulose E463 | 0.030 | 3.75 |
| Hydroxypropyl methylcellulose | 0.100 | 12.5 |
| Dibasic calcium phosphate dihydrate E 341 (Ii) | 0.2250 | 28.13 |
| Cross-linked sodium carboxymethylcellulose E 468 | // | // |
| Magnesium stearate (E470b) | 0.008 | 1 |
| Amorphous silica precipitated E 551 | 0.008 | 1 |

**Tablet 5 not coated** (batch MC024) and prepared as described in example 2.1 whose composition is reported in the following table 5:

**TABLE 5 -Composition of tablet 5**

| Components | g | % by weight |
|---|---|---|
| Palmitoylethanolamide (PEA) | 0.20202 | 25.25 |
| Vitamin B2 | 0.00182 | 0.23 |
| Microcrystalline cellulose - E 460 (i) | 0.20016 | 25.02 |
| Hydroxypropylcellulose E463 | 0.030 | 3.75 |
| Hydroxypropyl methylcellulose | 0.150 | 18.75 |
| Dibasic calcium phosphate dihydrate E 341 (Ii) | 0.2 | 25 |
| Cross-linked sodium carboxymethylcellulose E 468 | // | // |
| Magnesium stearate (E470b) | 0.008 | 1 |
| Amorphous silica precipitated E 551 | 0.008 | 1 |

**Tablet 6** (batch MC025) not coated prepared as described in example 2.1 whose composition is reported in the following table 6:

**Table 6 -Composition of tablet 6**

| Components | g | % by weight |
|---|---|---|
| Palmitoylethanolamide (PEA) | 0.20202 | 25.25 |
| Vitamin B2 | 0.00182 | 0.23 |
| Microcrystalline cellulose - E 460 (i) | 0.21516 | 26.90 |
| Hydroxypropylcellulose E463 | 0.030 | 3.75 |
| Hydroxypropyl methylcellulose | 0.100 | 12.5 |
| Dibasic calcium phosphate dihydrate E 341 (Ii) | 0.215 | 26.88 |
| Cross-linked sodium carboxymethylcellulose E 468 | // | // |
| Sodium alginate | 0.020 | 2.5 |
| Magnesium stearate (E470b) | 0.008 | 1 |
| Amorphous silica precipitated | 0.008 | 1 |
| E 551 | | |

**Tablet 7** not coated (batch MC027) prepared as described in example 1

**Table 7-Composition of tablet 7**

| Components | g | % by weight |
|---|---|---|
| Palmitoylethanolamide (PEA) | 0.20202 | 25.25 |
| Vitamin B2 | 0.00182 | 0.23 |
| Microcrystalline cellulose - E 460 (i) | 0.21516 | 26.90 |
| Hydroxypropylcellulose E463 | 0.030 | 3.75 |
| Hydroxypropyl methylcellulose | 0,075 | 9.38 |
| Dibasic calcium phosphate dihydrate E 341 (II) | 0.215 | 26.86 |
| Cross-linked sodium carboxymethylcellulose E 468 | // | // |
| Sodium alginate | 0.045 | 5.63 |
| Magnesium stearate (E470b) | 0.008 | 1 |
| Amorphous silica precipitated E 551 | 0.008 | 1 |

**Tablet 8** (batch MC031) coated with the coatings β1 and β2, respectively as described in example 2.1, whose composition is reported in the following table 8

**Table 8-Composition of tablet 8**

| | Components | g | % by weight |
|---|---|---|---|
| | Palmitoylethanolamide (PEA) | 0.20202 | 25.25 |
| **Core α)** | | | |
| | Vitamin B2 | 0.00182 | 0.23 |
| | Microcrystalline cellulose - E 460 (I) | 0.281 | 35.13 |
| | Hydroxypropylcellulose E463 | 0.055 | 6.88 |
| | Dibasic calcium phosphate dihydrate E 341 (Ii) | 0.24216 | 30.25 |
| | Cross-linked sodium carboxymethylcellulose E 468 | // | // |
| | Magnesium stearate (E470b) | 0.009 | 1.13 |
| | Amorphous silica precipitated E 551 | 0.009 | 1.13 |
| **Total** | | 0.800 | 100 |
| **Coating β1)** | polyvinyl alcohol, PEG + Macrogol, calcium carbonate * | 0.024 | 3% (weight increase) |
| **Coating β2)** | Sodium alginate and purified stearic acid ** | 0.00309286 | 0.38% (based on the total weight of the tablet with first coating) |
| | Ethylcellulose, medium chain fatty acid triglycerides, oleic acid*** | 0.01750714 | 2.12% (based on the total weight of the tablet with first coating) |

**Tablet 9 (Batch MC033)** coated with the coatings β1 and β2 respectively, prepared as described in example 2.1 and whose composition is reported in the following table 9

**Table 9- Composition of tablet 9**

| | Components | g | % by weight |
|---|---|---|---|
| **Core α)** | Palmitoylethanolamide (PEA) | 0.20202 | 25.25 |
| | Vitamin B2 | 0.00182 | 0.23 |
| | Microcrystalline cellulose - E 460 (i) | 0.265 | 33.13 |
| | Hydroxypropylcellulose E463 | 0.055 | 6.88 |
| | Dibasic calcium phosphate dihydrate E 341 (Ii) | 0.24216 | 30.27 |
| | Cross-linked sodium carboxymethylcellulose E 468 | 0.016 | 2 |
| | Magnesium stearate (E470b) | 0.009 | 1.13 |
| | Amorphous silica precipitated E 551 | 0.009 | 1.13 |
| **Total** | | 0.800 | 100 |
| **Coating β1)** | polyvinyl alcohol, PEG + Macrogol, calcium carbonate * | 0.024 | 3% (weight increase) |
| **Coating β2)** | Sodium alginate and purified stearic acid ** | 0.00309286 | 0.38% (on the total weight of the tablet with first coating) |
| | Ethyl cellulose, medium chain fatty acid triglycerides, oleic acid*** | 0.01750714 | 2.12% (on the total weight of the tablet with first coating) |

The friability and disintegration tests carried out with tablet 1 of the invention in comparison with tablets 2-9 are reported in the following table 10.

**Table 10**

| | Disint. | | | | | | Acceptabl e max value 1% | | | |
|---|---|---|---|---|---|---|---|---|---|---|
| Tablet No. (BATCH) | pH 6.8 (1h) | pH 6.8 | pH 1.1 (2h) | pH 1.1 (2h) + pH 6.8 | Hard ness (Kg) | Thick ness (mm) | Friability 4 min (%) | Friabili ty (24 mm) (%) | Abra sion (4 mm) (%) | Abrasi on (24 mm) (%) |
| 2 (FL749) | Test not performe d | Test not performed | Test not performed | Test not performe d | 15-18 | 5.8-6.0 | 0.30 | 1.57 | 0.14 | 0.55 |
| 3 (MC022) | The core remains intact, there is little powder. The centre of the core is dry | After 3 hours, the core slightly stains the buffer solution, but it is still intact. After 5 hours, the centre is still hard, although most disintegrate d | After 1 hour, the core almost completely disintegrated | Test not performe d | 14 | 6.2-6.25 | 0.10 | 1.04 | 0.06 | 0.18 |
| 4 (MC023) | The tablet remains intact, the water is | Test not performed | The tablet is intact, it slightly stains the buffer | After one hour in buffer at pH 6.8 it | 14-15 | 6.2-6.25 | 0.07 | 0.77 | 0.09 | 0.23 |
| | lightly stained . The centre of the core is dry | | solution. The centre of the core remains dry | is still intact, although it has lost little powder on the bottom. After 2 hours it lost more powder on the bottom and decreased in size, but the centre of the core remains hard, after 4 hours it has greatly decreased in size but the centre is still dry | | | | | | |
| 5 (MC024) | Intact, almost transpare nt water | After two hours it is still intact | Test not performed | Test not performe d | 14-15 | 6.4 | 0.09 | 0.84 | 0.06 | 0.16 |
| 6 (MC025) | Test not performe d | Test not performed | Intact, it slightly stains the solution at pH 1.1. The core is dry and does not lose volume | After 1 hour, it slightly stains the buffer solution, but it does not disintegra te | 14-15 | 6.25-6.3 | 0.19 | 1.18 | 0.18 | 0.38 |
| 7 (MC027) | intact | After more than 3 hours it is still intact | The tablet slightly stains the water but the centre of the core remains dry and solid | After 1 hour, the tablet did not disintegra te. | 14-15 | 6.15-6.20 | 0.14 | 0.94 | 0.11 | 0.30 |
| | | | | Core still dry after 3 hours | | | | | | |
| 8 (MC031) | intact | Intact | Intact | After 1 hour the tablet lost volume, but the centre of the dry core persists. | 14-16 | 6.15-6.20 | 0.17 | 0.94 | 0.18 | 0.42 |
| | | | | After 3 hours the tablet lost most of the volume, it reaches a thickness of about 1 mm. Disintegr ates within 210 minutes | | | | | | |
| **1 (MC032)** | **The tablet has partially disintegr ated and has stained the water** | **Over 1.5 hours the tablet completely disintegrate d.** | **The tablet completely disintegrated after 1.5 hours.** | **Test not performe d** | **15-16** | **6.15** | **0.16** | **0.89** | **0.14** | **0.29** |
| | | **The water is yellow** | | | | | | | | |
| 9 (MC033) | Over 15 minutes, the tablet is totally disintegra ted | Test not performed | Test not performed | Test not performe d | 14-15 | 5.8 - 6.1 | 0.12 | 0.89 | 0.12 | 0.32 |

From the results reported in the aforesaid table it can be seen how only tablet 1 according to the present invention meets the optimal parameters in terms of friability and disintegration of the tablet only enterically.

## Claims

1. Gastro-resistant tablet comprising a core α) and a gastro-resistant coating β), wherein said core α) comprises or consists of:
a) Palmitoylethanolamide;
b) at least one diluent selected from: Microcrystalline cellulose and pregelatinized starch
c) a binding agent consisting of hydroxypropyl cellulose;
d) at least one inorganic filler selected from: Dibasic calcium phosphate dihydrate, or calcium carbonate;
e) at least one lubricating agent selected from magnesium stearate, precipitated amorphous silica or mixtures thereof
f) a disintegrating agent consisting of cross-linked sodium carboxymethylcellulose, and
**wherein**
I) the palmitoylethanolamide is not in an ultra-micronized form nor is it in a micronized form
II) the weight ratio of sodium hydroxypropyl cellulose to carboxymethylcellulose is between 4.5 and 10.

2. Tablet according to claim 1, wherein said weight ratio II) is 6.9.

3. Tablet according to claim 1 or 2, wherein the palmitoylethanolamide a) is present at concentrations comprised between 23 and 27%, preferably at a concentration of 25.25% by weight on the total weight of said core by weight.

4. Tablet according to any one of claims 1-3, wherein the diluent b) is microcrystalline cellulose and is present at concentrations comprised between 30 and 40%, preferably equal to 34.13%, by weight on the total weight of said core.

5. Tablet according to any one of claims 1-4, wherein the hydroxypropylcellulose or binding agent c) is present at concentrations comprised between 5 and 7.5%, preferably above 6.5%, more preferably equal to 6.88% by weight on the total weight of said core.

6. Tablet according to any one of claims 1-5, wherein the filler agent d) is dibasic calcium phosphate and is present in concentrations comprised between 25 and 35%, more preferably equal to 30.27% by weight on the total weight of said core.

7. Tablet according to any one of claims 1-6, wherein the lubricating agent e) consists of a mixture of magnesium stearate and amorphous silica precipitated at total concentrations comprised between 2 and 3%, preferably equal to 2.26% by weight on the total weight of said core.

8. Tablet according to any one of claims 1-7, wherein the cross-linked sodium carboxymethylcellulose or disintegrating agent f) is in a concentration comprised between 0.5 and 3%, preferably equal to 1% by weight on the total weight of the core.

9. Tablet according to any one of claims 1-8, wherein the core (α) is coated with a coating β) comprising:
- a first opacifying coating β1) which wraps the aforesaid core comprising or consisting of:
g) at least one film-forming polymer consisting of polyvinyl alcohol
h) an opacifying agent selected from calcium carbonate, talc and mixtures thereof;
i) at least one plasticizer selected from polyethylene glycol, macrogol or mixtures thereof;
- a second gastroprotective coating β2 wrapping the tablet coated with the coating β1, said second coating comprising:
j) a film-forming polymer selected from ethylcellulose and propyl cellulose:
k) a plasticizer selected from a mixture of medium chain fatty acid triglycerides; oleic acid or mixtures thereof;
l) a lubricant selected from stearic acid and its salts
m) a thickening agent selected from sodium alginate, gum arabic

10. Tablet according to claim 9, wherein the coating β1constitutes between 2.5 and 3.5%, preferably 3% by weight on the total weight of the core (α) and the coating β2 constitutes between 2 and 4%, preferably 2.5% by weight on the total core weight.

11. Tablet according to claims 8 or 9, wherein, in the first coating:
- the at least one film-forming polymer g) is polyvinyl alcohol at concentrations comprised between 38% and 42%, more preferably 40% by weight on the total weight of said first coating;
- the opacifying agent h) consists of a mixture of calcium carbonate and talc at concentrations comprised between 38% and 42%, more preferably 39.8% by weight on the total weight of said first coating;
- the plasticizer i) is a mixture of PEG and Macrogol at concentrations comprised between 18% and 22%, more preferably 20.20% by weight on the total weight of said coating.

12. Tablet according to any one of claims 9-11, wherein in the second coating:
- the film-forming polymer j) ethyl cellulose is present at concentrations comprised between 17.5% and 19.5%, more preferably actually 18.80% by weight on the total weight of said coating;
- the plasticizer k) is a is a mixture of triglycerides of medium chain fatty acids and oleic acid at concentrations comprised between 3% and 5%, more preferably 4% by weight on the total weight of said second coating
- the lubricant l) is stearic acid at concentrations preferably comprised between: 0.03 and 0.05%, more preferably 0.0423% by weight on the total weight of said second coating:
- the thickening agent m) is sodium alginate at concentrations comprised between 3.5% and 5.5%, more preferably 4.18% by weight on the total weight of said coating.

13. Tablet according to any one of the preceding claims, comprising Vitamin B2 dispersed in the core α) as a further ingredient to promote intestinal barrier integrity.

14. Process for preparing tablets according to any one of claims 9-13, comprising the following steps:
i) The components a) -f) are dry mixed;
ii) the mixture from the previous step is compressed;
iii) a suspension in water and/or ethanol of components g), h) and i) is prepared so as to have a total concentration of components g) + h) + i) comprised between 15% and 35%, more preferably 20.0% by weight on the total weight of said suspension in water and/or ethanol;
iv) applying the coating β1) by nebulising the suspension at a temperature comprised between 38°C and 46°C until the suspension to be nebulized is exhausted;
v) the coated tablet from step iv) is cooled;
vi) the aqueous suspension of components j), k), l), and m) is prepared until a total concentration of j) +k) + l) + m) is obtained between 8% and 15%, more preferably 10% by weight on the total weight of the aqueous suspension;
vii) the coating β2) is applied by nebulisation of the aqueous suspension obtained in step
vi) at a temperature comprised between 38°C and 46°C, and is maintained at such a temperature until the suspension to be nebulized is exhausted.
